# EUROPEAN PATENT APPLICATION

(11) **EP 2 545 910 A1**
(43) Date of publication of application: **16.01.2013**
(21) Application number: 11173621.1
(22) Date of filing: 12.07.2011
(51) Int. Cl.: A61K 31/00, A61K 31/7004, A61K 31/7016, A61P 35/00

(54) **Use of Lipoteichoic Acid in the treatment of cancer in an inflammatory state**

(71) Applicant: Lunamed AG, 7000 Chur (CH)
(72) Inventor: Truog, Peter, 7000 Chur (CH)
(74) Representative: Peters, Hajo

(57) **Abstract**

The present invention refers to the treatment of pro inflammatorically treated cancer in an inflammatory state using Lipoteichoic acid especially also to a treatment of cancer previously treated with radiation or with pro-inflammatorically active substances.

## Description

### Field of the invention

The present invention refers to the treatment of pro inflammatorically treated cancer in an inflammatory state using Lipoteichoic acid especially also to a treatment of cancer previously treated with radiation or with pro-inflammatorically active substances.

### Background of the invention

Cancer is a one of the most dreaded and feared diseases capturing the intense interest of nearly all levels of society but also causing enormous cost to health care. It is one of the most common causes of death worldwide. The efforts to fight or to contain cancer are considerably and thus treatment of cancer is one of the most important targets of modern medicine. Even though a lot of medical treatments have been presented so far and over the last decades, with not a few of them with extreme grave consequences to the patient, there is still a big need for a treatment going beyond the usual treatment known today ameliorating it effect and minimizing the side effects. An especially excruciating practice is treatment of an identified tumor or the tissue surrounding a surgically removed tumor by radiation. The clear logic to this treatment being the hoped-for effect that the tissue of the tumor or any remaining cancerous cells possibly still existing in the surrounding of the extracted tumor would be destroyed by this harsh treatment. Still, even though undeniably often successful to some degree unfortunately this treatment - causing heavy inflammatory in the radiated tissue - also often not suffice to remove the cancer or its risk of recurrence.

Thus a central goal of research and of this invention was to provide means for cancer treatment going beyond or ameliorating the existing treatments, especially avoiding the or some disadvantages accompanying this or these treatments thus providing an improved way of treating cancer. Such improvements would include improving the clinical results seen in patients treated after suffering from cancer such as prolongation of survival time, reduction or even removal of cancer cells, allowing patients to be effectively treated that were not or only to a limited degree treatable before, reducing side-effects seen in patients suffering from cancer or increasing or decreasing levels of biomarkers used as parameters to indicate success in such a cancer treatment with these improvements being shown either in one or more of the above listed aspects. Such an improvement could further also include a reduction in dose necessary, reducing the number of application per day in parallel standard treatments or reducing side effect of such a parallel standard treatment. Such an improvement could further also result in an increase of compliance in a patient treated for suffering from cancer.

This invention surprisingly identified a way to fulfill this goal by the treatment of cancer in an inflammatory state using Lipoteichoic acid.

Accordingly, the invention is drawn to lipoteichoic acid as free acid or as any pharmaceutically acceptable salt or hydrate thereof for use in the treatment of cancer being in an inflammatory state. This would thus also encompass lipoteichoic acid as free acid or as any pharmaceutically acceptable salt or hydrate thereof for use in the treatment of cancer after induced inflammation in the cancer. And this would even encompass lipoteichoic acid as free acid or as any pharmaceutically acceptable salt or hydrate thereof for use in the treatment of pro-inflammatorically co-treated cancer.

In a parallel way the invention is thus also drawn to a method of treatment of cancer being in an inflammatory state by providing to a patient in need thereof a sufficient amount of lipoteichoic acid as free acid or as any pharmaceutically acceptable salt or hydrate thereof. This would thus also encompass a method of treatment of cancer by providing to a patient in need thereof after induced inflammation in the cancer a sufficient amount of lipoteichoic acid as free acid or as any pharmaceutically acceptable salt or hydrate thereof. And this would even encompass a method of treatment of pro-inflammatorically co-treated cancer by providing to a patient in need thereof a sufficient amount of lipoteichoic acid as free acid or as any pharmaceutically acceptable salt or hydrate thereof. All the following features and embodiment disclosed for the lipoteichoic acid for the use in cancer, embodiments of the invention relating to lipoteichoic acid etc. are also applicable to these methods of treatment. Thus also a method of treatment of pro-inflammatorically co-treated cancer by providing to a patient in need thereof a sufficient amount of lipoteichoic acid as free acid or as any pharmaceutically acceptable salt or hydrate thereof in which the pro-inflammatoric co-treatment is done by (X-ray) (ionizing) radiation is (and would be thus) disclosed. Thus this invention also includes a method of treatment of cancer by providing to a patient which had undergone an ionizing X-ray radiation (possibly following surgical removal of a tumor) a sufficient amount of lipoteichoic acid as free acid or as any pharmaceutically acceptable salt or hydrate thereof to treat the remaining cancer/tumors/cancer cells in the tissue having undergone radiation. This treatment with lipoteichoic acid could then be done by applying (injecting) e.g. subcutaneously or intra-abdominally (e.g. 1 µmol) lipoteichoic acid (LTA-T) in one or more applications per day.

Lipoteichoic acid (LTA) is a gram positive pathogen cell wall motif that allows the pathogen to be immunologically recognised by binding to Toll-like receptors on the cell surface. LTAs have been reported as having anti tumor activity (EP 135820; USP 4,678,773; A. Yamamoto et. al. 1985, Br. J. Cancer, 51, 739 - 742; and H. Usami et. al., Br. J. Cancer, 1988, 57, 70 - 73) ansd especially also WO 96/023896.

Even though the use of LTA in cancer was thus known in the art, it came as a very positive surprise to find out that tissue containing cancer cells and cancerous tissue lumps like already encapsulated tumors which were pre-treated in a pro-inflammatory way, e.g. inside the focus of a preceding radiation treatment, disappeared/were found to have been removed when a thus treated patient was given LTA. This effect was impressive, especially by comparison to the removal rate of cancerous tissue outside the focus of this radiation in the thus treated patient having received LTA where especially encapsulated tumors remained often intact and unaffected by LTA.

### Detailed Description:

Thus, the invention relates to lipoteichoic acid as free acid or as any pharmaceutically acceptable salt or hydrate thereof for use in the treatment of cancer being in an inflammatory state, with this inflammation preferably being local. This would thus also encompass lipoteichoic acid as free acid or as any pharmaceutically acceptable salt or hydrate thereof for use in the treatment of cancer after induced inflammation in the cancer. This would also encompass lipoteichoic acid as free acid or as any pharmaceutically acceptable salt or hydrate thereof for use in the treatment of pro-inflammatorically treated cancer.

"Cancer being in an inflammatory state" is defined as a state of the cancer, tumor or the cancer cells in that local inflammation is directly affecting the cancer, tumor or the cancer cell itself or the tissue immediately surrounding these cells. It is the state in which inflammatory mediators are released into or existing in the cancer, the tumor or the cancer or tumor cells or the tissue immediately surrounding these cells thus showing signs of inflammation.

"After induced inflammation in the cancer" is defined as treatment at any time, e.g. within 1 or 2 weeks after any event that induces inflammation in a cancer, meaning within at least a part of the cancer cells to be treated. Events that could induce inflammation in the cancer to be treated could include (local) inflammatory reactions of the body, or especially pro-inflammatory treatment of the cancer to be treated (or the patient suffering from this cancer) including radiation treatment or application (like injection) of pro-inflammatory substances e.g. locally into the tumor or tissue surrounding it Often this "induced inflammation in the cancer" is a local event or locally induced (e.g. through local pro-inflammtory treatment, radiation/injection).

"Pro-inflammatorically treated cancer" is defined as a cancer (tumor or cancer cell) that has been (previously/before the treatment with LTA , e.g. within 1 or 2 weeks before) treated (or the patient suffering from this cancer) in any way to induce inflammation in the cancer to be treated. One example of such a treatment resulting in "pro-inflammatorically treated cancer" would be radiation treatment of the cancer or any tissue comprising cancer (tumors/cancer cells) like an identified tumor or the tissue surrounding the excision area after tumor removal correctly suspected of still comprising cell-lumps or tumors. One other example is the application of pro-inflammatory substances (e.g. by injection) to an identified tumor or the tissue surrounding the excision area after tumor removal. Often this treatment resulting in pro-inflammatorically treated cancer induced to inflammation is a local treatment opposed to a systemic treatment.

In a preferred embodiment of the invention relating to lipoteichoic acid as free acid or as any pharmaceutically acceptable salt or hydrate thereof for use in the treatment of cancer being in an inflammatory state, the treatment is done after induced inflammation in the cancer, preferably after locally induced inflammation in the cancer. Preferably the induced inflammation is done by pro-inflammatorical co-treatment of the cancer, preferably by local pro-inflammatorical co-treatment of the cancer.

Inside this common focus, the invention thus also relates to lipoteichoic acid as free acid or as any pharmaceutically acceptable salt or hydrate thereof for use in the treatment of cancer after induced inflammation in the cancer. Preferably the induced inflammation is done by pro-inflammatorical co-treatment of the cancer, preferably by local pro-inflammatorical co-treatment of the cancer.

Inside this common focus, the invention thus also relates to lipoteichoic acid as free acid or as any pharmaceutical acceptable salt or hydrate thereof for use in the treatment of pro-inflammatorically co-treated cancer. Preferably the inflammation or the co-treatment inducing such inflammation is local.

"Local" is defined here as in the immediate area (or tissue) around the cancer (the tumor or cancer cell) to be treated. Especially regarding treatment it is defined as being non-systemic but instead being applied to the immediate area (or tissue) surrounding the cancer (the tumor or cancer cell) to be treated or to the cancer itself, e.g. by injection or topically etc.

It is very important to note that in all aspects and features in which a "local" treatment is described it is also in certain circumstances very preferred to use systemic treatment instead of local treatment. This especially applies to the treatment with lipoteichoic acid but also especially to the application of at least one pro-inflammatory substance which could also be applied systemically. Thus, anytime "local" treatment is disclosed it should be understood that the opposite "systemic" treatment is disclosed for the treatment as well.

In a preferred embodiment the treatment is following while the cancer is in a state of inflammation.

In another a preferred embodiment the treatment is following within 1 or 2 months, or within 1 or 2 weeks or within 1 week, or within 1 to 5 days, or within 1 to 3 days after induction of inflammation in the cancer or after pro-inflammatorical co-treatment of the cancer.

In a preferred embodiment the pro-inflammatorical co-treatment is done by radiation or by application of at least one pro-inflammatory substance.

In a preferred embodiment the pro-inflammatorical co-treatment is done by radiation, preferably by radiation of the tissue comprising the cancer, preferably by X-ray radiation of the tissue comprising the cancer. Preferably, this radiation (the co-treatment) is done "previously", meaning before the treatment with lipoteichoic acid, but there might also be cases in which the lipoteichoic acid treatment is already applied or is even done simultaneously with the radiation co-treatment.

"Radiation" (treatment) is defined as treatment of tissue comprising or having comprised cancer or a tumor or cancer cells by radiation, preferably by X-Ray radiation in an intensity intended on the destruction of the cancer, the tumor or the cancer cell or change the genes to inhibit growth. This treatment causes inflammation in the cancer, tumor or cancer cells or in the tissue immediately surrounding the cancer/tumor or the cancer cells. Specifically this is ionizing radiation as ions are formed in the cells of the tissues it passes through.

In a preferred embodiment the pro-inflammatorical co-treatment is done, optionally previous to the treatment, by application of at least one pro-inflammatory substance, preferably is done, optionally previous to the treatment, by application of at least one pro-inflammatory substance to the tissue comprising the cancer and/or is done, optionally previous to the treatment, by injection of at least one pro-inflammatory substance, more preferably is done, optionally previous to the treatment, by injection of at least one pro-inflammatory substance to the tissue comprising the cancer. By "optionally previous to the treatment" is meant that the application of at least one pro-inflammatory substance is - often or in many circumstances even preferredly - done previous to the treatment with lipoteichoic acid. Still, there are or might also be cases in which the lipoteichoic acid treatment is already applied or is even done simultaneously (even e.g. in one injection) with the co-treatment by application of at least one pro-inflammatory substance. In a preferred embodiment the pro-inflammatory substance is selected from pro-inflammatory interleukins.

"Pro-inflammatory substance" is defined as a substance causing inflammation in the tissue (being it cancer, tumor or cancer cells) or the tissue surrounding those. Examples include the ever-spreading group of pro-inflammatory interleukins.

In a very preferred embodiment of the lipoteichoic acid according to the invention the treatment with lipoteichoic acid is done by injection, including for example s.c. (subcutaneous), i.m. (intramuscular), i.v. (intravenous), i.a. (intra-arterial), intra-ocular, intra-pleural, intra-abdominal, or intra-thecal injection.

In a very preferred embodiment of the lipoteichoic acid according to the invention the treatment with lipoteichoic acid is done by applying an amount of 0.1 to 10 µmol, 0.5 to 2.0 µmol or 1 µmol lipoteichoic acid applied in one or several applications per day.

Preferably the treatment with the lipoteichoic acid is done by injection (for example s.c., intra-pleural, intra-abdominal, or intra-thecal injection) of 0.5 to 2.0 µmol or 1 µmol of lipoteichoic acid (e.g. in 1µmol/ml). Preferably the lipoteichoic acid in such an injectable formulation is preferably dissolved in a physiologically acceptable aqueous medium, most preferably in physiological saline (NaCl 0.9%). The injectable formulation may be used for single or repeated (from 1 to 10 times per day) injections.

The medicament or pharmaceutical formulation for use in the treatment of the present invention may thus in general be administered parentally in combination with conventional injectable liquid carriers, such as water or suitable alcohols (injectable formulations). Conventional pharmaceutical excipients for injection, such as stabilizing agents, solubilizing agents, and buffers, may be included in such injectable compositions.

In general though, the treatment with the lipoteichoic acid can be done with any pharmaceutical formulation or medicament comprising lipoteichoic acid and optionally one or more pharmaceutically acceptable excipients. The medicament or pharmaceutical formulation for use in the treatment according to the present invention can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

Medicaments or pharmaceutical formulation for use in the treatment according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation for use in the treatment according to the present invention may also comprise an enteric coating, so that their dissolution is dependent on pH-value. Due to said coating the medicament may pass the stomach undissolved and the respective pharmaceutical composition according to the central aspect of the invention is liberated in the intestinal tract. Preferably the enteric coating is soluble at a pH value of 5 to 7.5. Suitable materials and methods for the preparation are known from the prior art. Typically, these medicaments for use in the treatment according to the present invention may contain 1-60 % by weight of lipoteichoic acid and 40-99 % by weight of one or more auxiliary substances (additives/excipients).

The pharmaceutical combination for use in the treatment of the present invention or medicament or pharmaceutical formulation for use in the treatment of the invention may also be administered topically or via a suppository.

"Lipoteichoic acids" - also abbreviated as "LTA" used herein is an inclusive term including the a) lipoteichoic acids (LTA) There are many lipoteichoic acids known which form a major part of the cell wall of gram-positive bacteria. The known lipoteichoic acids include synthetic, semisynthetic and naturally occurring lipoteichioic acids. A very broad introduction on the synthesis of as well as on synthetic lipoteichoic acids can be found in Pedersen, Christian Marcus; Schmidt, Richard R. (Edited by Moran, Anthony P), Microbial Glycobiology (2009), 455-476 with all lipoteichoic acids described therein herewith included by reference in this application as examples of the lipoteichoic acids to be used. A further overview of the lipoteichoic acids - divided in Types I to IV - can be found in Greenberg et al., Infect Immun. 1996 Aug;64(8):3318-25. All lipoteichoic acids Type I, Type II, Type III and Type IV as well as all those described therein (like e.g. in tables 1 or 2) herewith included by reference in this application as examples of the lipoteichoic acids to be used. LTAs were isolated from e. g. Lactobacillus helveticus (NCIB 8025), Lactobacillus fermenti (NCTC 6991), Streptococcus faecalis, 39, Streptococcus lactis (ATCC 9936), Streptococcus mutans, AHT (A. J. Wicken et al, 1975), and Streptococcus pyogenes SV strain (ATCC 21059) (EP 135 820, USP 4,678,773, H. Usami et. al. 1985).

In a preferred embodiment of the lipoteichoic acid for use according to the invention the lipoteichoic acid is a (general) lipoteichoic acid. The (general) lipoteichoic acids as defined in this application are comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

Lipoteichoic acids falling under this definition according to this invention are called "(general) lipoteichoic acids" especially hereinafter.

The glycolipid acts as an anchor in the cell membrane. The glycolipids are by definition of the invention lipids of the membrane in which one or more mono- or oligosaccharids are bound via a glycosidic bond to a lipid molecule. The lipid molecule is consisting of fatty acids being bound by an ester bond to a glycerol or by an amide bond to a sphingosine.

In one preferred embodiment of the lipoteichoic acid for use according to the invention relating to a (general) lipoteichoic acid the glycolipid is a glyceroglycolipid in which one or more mono- or oligosaccharids are bound via a glycosidic bond to a lipid molecule consisting of fatty acids being bound by an ester bond to the glycerol. Preferably the glyceroglycolipid comprises a diacylglycerol with two fatty acid chains of 10 to 20 carbon atoms each being bound by an ester bond to the glycerol; and/or preferably the fatty acid chains are linear or branched and non-substituted and/or have a length of 10 to 20 carbon atoms each, most preferably 12, 14, 16 or 18 carbon atoms.

Most (general) lipoteichoic acids would be covered by the general formula I and its substituents as described and defined below and some preferred embodiments are described below, but some are unusual. These unusual include the lipoteichoic acid from *Streptococcus pneumoniae* (see below) whose synthesis was recently described by Pedersen et al., in Angew. Chem. (2010), 122, 1 - 7. 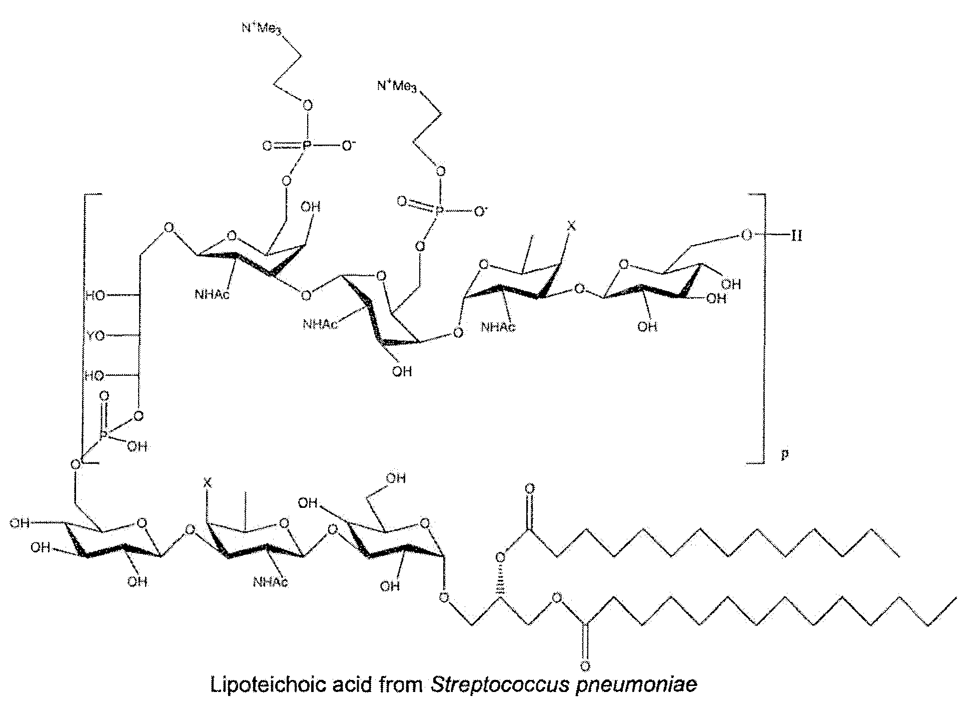 wherein
in one case X is NH₃⁺, or NHAc; Y is H, D-Ala, or α-GalNAc; and p is up to 8;
in the other case X is NH₃⁺; Y is H; and p is 1.

In a preferred embodiment of the lipoteichoic acid for use according to the invention the lipoteichoic acid is a lipoteichoic acid according to general formula I 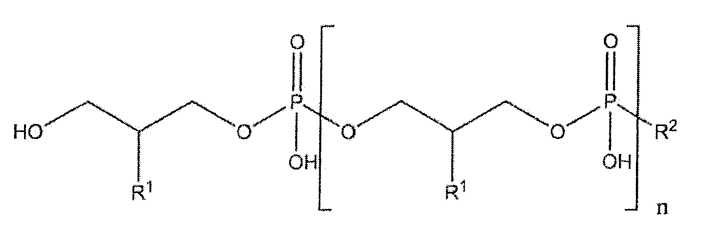 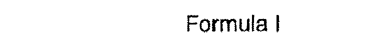 wherein
the mean value of n is 8 to 40,
R¹ is either OH or O-D-alanyl and
R² is
either 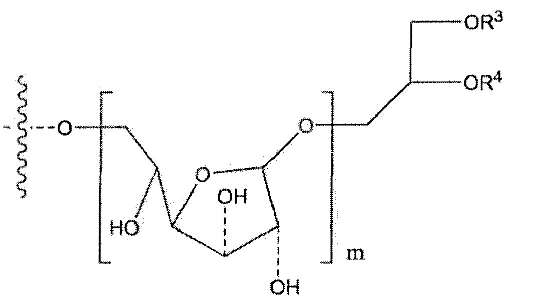 or its tautomeric equivalent 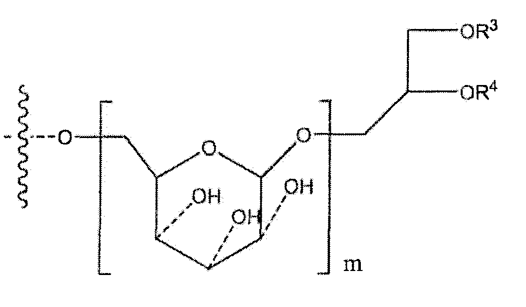 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

In one even more preferred embodiment of the lipoteichoic acid for use according to the invention the lipoteichoic acid is a lipoteichoic acid according to general formula I 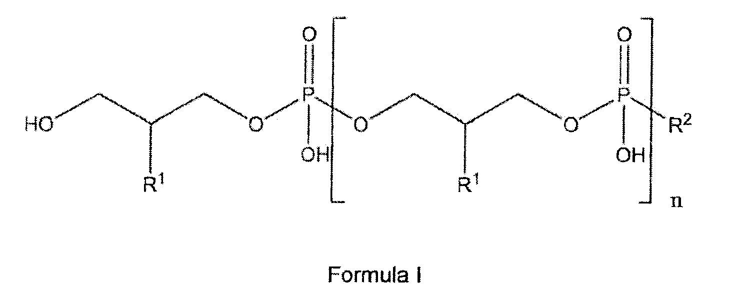 wherein
the mean value of n is 9,
R¹ is either OH or O-D-Alanyl and
R² is 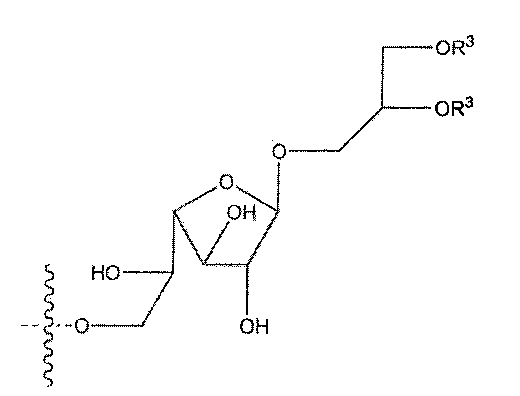 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures. These compounds defined above as well as those described by general formula Ia, lb, lc or Id and their defined radicals below (as well as any lipoteichoic acid to be isolated from the Streptococcus sp. PT strain DSM 8747) are defined in this invention as "lipoteichoic acid of the invention".

In a further embodiment of the lipoteichoic acid for use according to the invention the lipoteichoic acid is a purified lipoteichoic acid isolated or isolatable from the Streptococcus sp. PT strain DSM 8747, preferably containing a beta-galactofuranosyl(1-3)glycerol-di-ester moiety.

In a preferred embodiment of the lipoteichoic acid for use according to the invention the lipoteichoic acid is a lipoteichoic acid according to any of general formulas la, Ib, Ic or Id 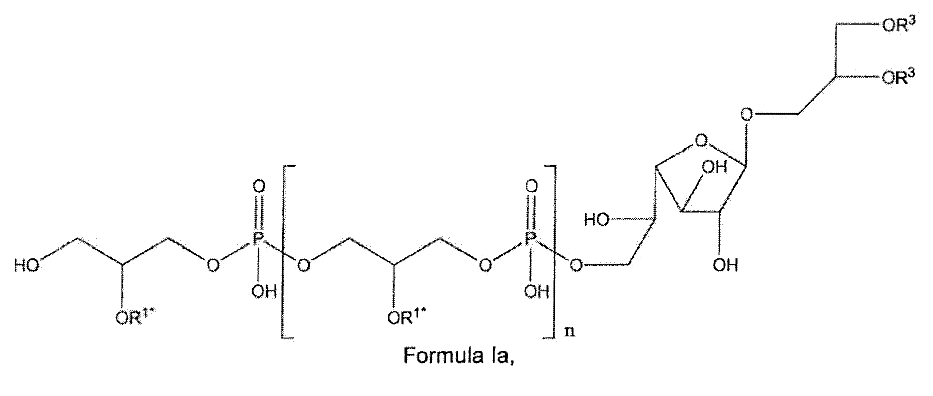 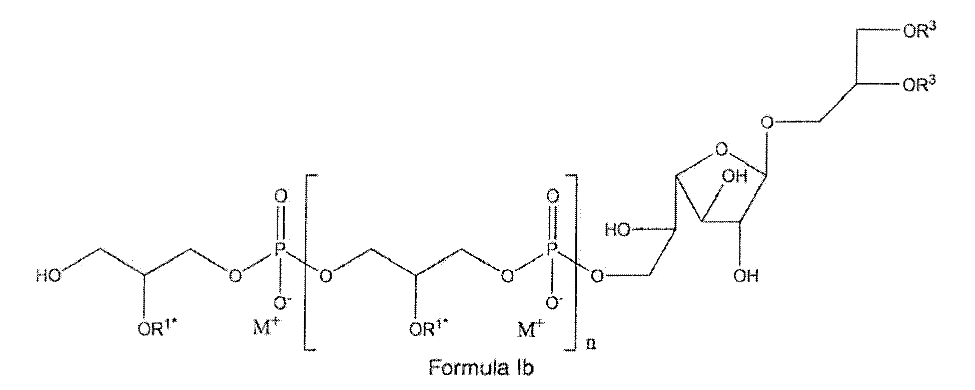 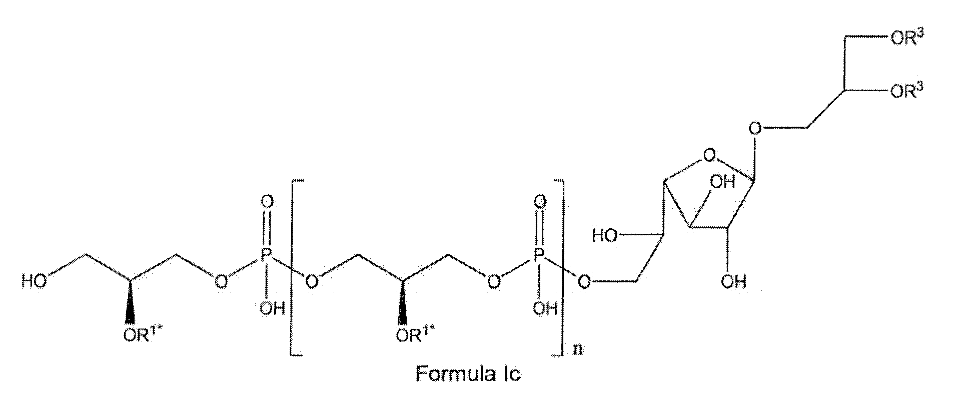 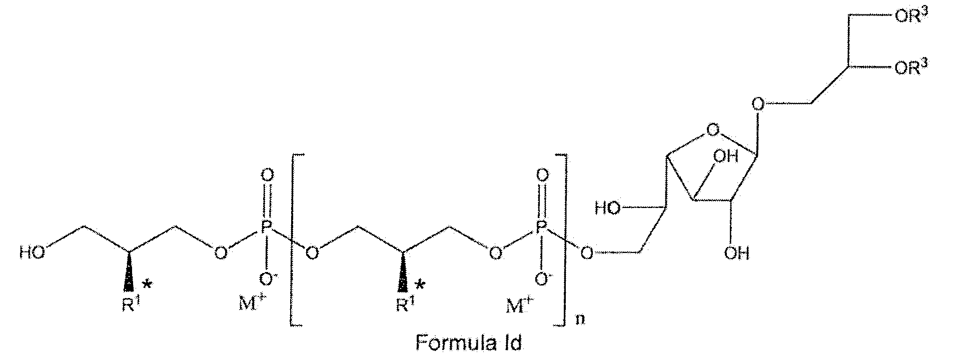 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

In a preferred embodiment of the lipoteichoic acid for use according to the invention the lipoteichoic acid is a purified lipoteichoic acid isolated from bacteria of the genus *streptococcus,* preferably from *streptococcus sp*., most preferably from *streptococcus sp.* (DSM 8747). One example of such an isolated lipoteichoic acid is lipoteichoic acid T (LTA-T).

Lipoteichoic acids - especially the lipoteichoic acid T (LTA-T) - are described in the PCT-application WO 96/23896 together with its antitumor cholesterol-lowering activity and ways of isolating the LTA-T. The whole disclosure of WO06/23896 is included in this application by way of reference.

LTA-T is a gram positive cell wall motif that allows it to be immunologically recognised by binding to Toll-like receptors on the cell surface. LTA-T is described in US 6,114,161 for use as an antitumour preparation. All aspects of LTA-T from US 6,114,161 are incorporated herein by reference.

Preferably, the "lipoteichoic acid" is lipoteichoic acid T (LTA-T). Thus, in one very preferred embodiment the lipoteichoic acid for use according to the invention is lipoteichoic acid T (LTA-T).

Lipoteichoic acid or especially lipoteichoic acid T (LTA-T) may form salts with other (positively) charged ions, in particular physiologically acceptable salts, such as alkali metal or alkaline earth metal salts, also heavy metal salts, such as zinc or iron salts, or primary, secondary, tertiary or quaternary ammonium salts (acid addition salts) . Such other salts are e. g. potassium, calcium, ammonium, mono-, di-, tri- or tetra-lower alkyl-, like methyl- or ethyl-, or methyl-ethyl, propyl- or butylammonium salts. A prefered salt of LTA-T is the sodium salt.

In another embodiment of lipoteichoic acid for use according to the invention the lipoteichoic acid according to general formula I is a lipoteichoic acid of *staphylococcus aureus* (below): 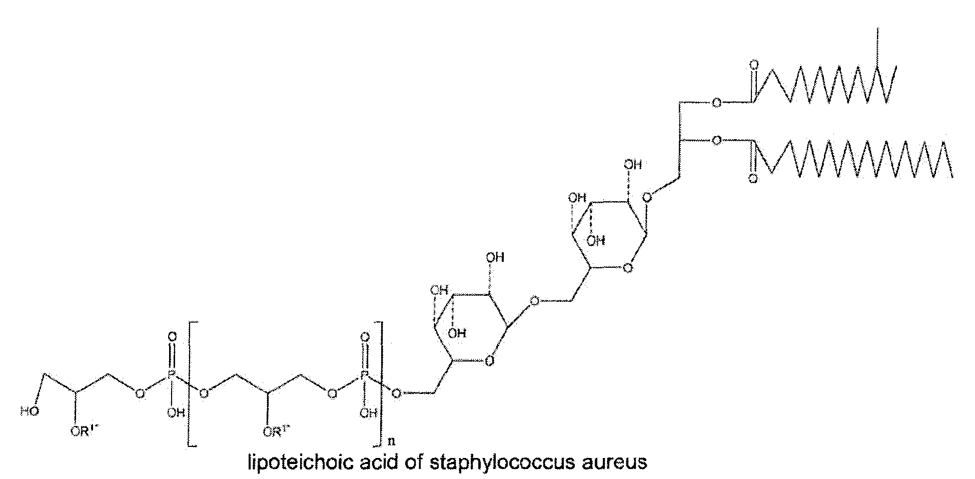 wherein X is H or D-ala and the mean value of "n" is 16 to 40.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In another preferred embodiment of the invention the treatment is done with a pharmaceutical composition comprising at least one lipoteichoic acid as free acid or as any pharmaceutically acceptable salt or hydrate thereof and at least one D-amino acid as free acid or as any pharmaceutically acceptable salt or hydrate thereof. Preferably this lipoteichoic acid is any of the lipoteichoic acids set out above and most preferably is LTA-T. The D-amino acid may be any D-amino acid but preferably is D-alanine. This pharmaceutical composition is preferably an injectable formulation comprising (e.g. 1 µmol) Lipoteichoic acid (LTA-T) and (e.g. 50 mg) D-amino acid (D-alanine).

This inventive concept would also apply to a related aspect of the invention wherein the treatment is done by applying (e.g by (e.g. s.c.) injection of an injectable formulation) a pharmaceutical composition comprising at least one lipoteichoic acid (LTA-T) as free acid or as any pharmaceutically acceptable salt or hydrate thereof and by applying separately (e.g orally in an oral pharmaceutical formulation) before or after or simultaneously at least one D-amino acid (D-alanine) as free acid or as any pharmaceutically acceptable salt or hydrate thereof. This related aspect would naturally be also the subject of a method of treatment of cancer.

One very preferred aspect of the invention would also encompass the lipoteichoic acid for the use according to the invention in which the cancer is in form of non-encapsulated cancer cells. This would thus also encompass a method of treatment or better of prophylaxis against (by lowering the risk of getting) cancer by providing to a patient in need thereof (at risk of suffering from cancer in the future (due to non-encapsulated cancer cells) or accordingly just having undergone a surgical cancer treatment or biopsy) a sufficient amount of lipoteicoic acid, in which the patient is further co-treated (like local treatment e.g. at or around the area of surgery of biopsy) in a pro-inflammatory way. This pro-inflammatory co-treatment might occur by (X-ray; iodizing) radiation or by application of at least one pro-inflammatory substance like pro-inflammatory interleukins. By this way the chances of a successful prophylactic treatment are increased.

"Non-encapsulated cancer cells" are cancer/neoplastic cells not (yet) encapsulated by a capsule like a hyaluronic acid capsule. This includes cells not accumulated or aggregated with other cancer cells or to only accumulated or aggregated small groups of cancer cells not yet encapsulated and would also include microtumors.

A last aspect of the invention would further encompass a method of treatment or better of prophylaxis against (by lowering the risk of getting) cancer by providing to a patient in need thereof (at risk of suffering from cancer in the future (due to non-encapsulated cancer cells) or accordingly just having undergone a surgical cancer treatment or biopsy) a sufficient amount of lipoteichoic acid, in which the patient is further co-treated (like local treatment e.g. at or around the area of surgery of biopsy) in a pro-inflammatory way and in which the treatment with lipoteichoic acid is done with a pharmaceutical composition comprising at least one lipoteichoic acid as free acid or as any pharmaceutically acceptable salt or hydrate thereof and at least one D-amino acid as free acid or as any pharmaceutically acceptable salt or hydrate thereof. Preferably this lipoteichoic acid is any of the lipoteichoic acids set out above and most preferably is LTA-T. The D-amino acid may be any D-amino acid but preferably is D-alanine. This pharmaceutical composition is preferably an injectable formulation comprising (e.g. 1 µmol) lipoteichoic acid (LTA-T) and (e.g. 50 mg) D-amino acid (D-alanine). The pro-inflammatory co-treatment might occur by (X-ray; iodizing) radiation or by application of at least one pro-inflammatory substance like pro-inflammatory interleukins.

The last aspect of the invention would also apply to a related aspect of the invention wherein the treatment is done by applying (e.g by (e.g. s.c.) injection of an injectable formulation) a pharmaceutical composition comprising at least one lipoteichoic acid (LTA-T) as free acid or as any pharmaceutically acceptable salt or hydrate thereof and by applying separately (e.g orally in an oral pharmaceutical formulation) before or after or simultaneously at least one D-amino acid (D-alanine) as free acid or as any pharmaceutically acceptable salt or hydrate thereof. This last aspect would naturally be also the subject of a method of treatment of cancer.

The present invention is illustrated below with the help of the following examples. These illustrations are given solely by way of example and do not limit the invention.

### EXAMPLES:

### Example 1: Pharmaceutical Formulation

The purified LTA-T in 0.05M sodiumacetate pH 4.7 (prepared as set out in WO 1996/023896) is dialysed extensively against physiological NaCl solution (0.9%) and the volume is adjusted to 1 µmol LTA-T (based on the phosphorus content) within 1 ml of physiological saline. After filtration of the solution through a filter membrane (Millipore 0.22um), 1 ml aliquots of the filtrate are placed in sterilized vials under sterile conditions. These vials contain 1 micromol/ml LTA-T phosphorus and are used subcutaneously for therapeutical purposes.

### Example 2: Treatment of a patient with recurrent carcinoma in irradiated field

A female patient I.S. born 1926 was diagnosed with breast carcinoma. She was treated by ablation of the left breast. The pathology was showing that invasive ductal carcinoma with 7/7 axillary lymph nodes positive. More than 6 years after treatment a local recurrence in left thoracic skin, proven by histological examination was found. The left thoracic wall was treated by X-ray therapy with 60 gray (local boost axilla and supra-clavicular area with 60.4 gray). More than 3 years later osteoplastic bone metastases and 6 months later extensive local recurrence in the irradiated field of left thoracic wall were diagnosed. 6 months later the patient was treated by s.c injections of 1 µmol of LTA-T in 1 ml physiological saline (according to example 1) over a period 6 weeks. The result was a clinically and histologically complete remission within the irradiated field.

## Claims

1. Lipoteichoic acid as free acid or as any pharmaceutically acceptable salt or hydrate thereof for use in the treatment of cancer being in an inflammatory state.

2. The Lipoteichoic acid according to claim 1 wherein the treatment is done after induced inflammation in the cancer, preferably after locally induced inflammation in the cancer.

3. The Lipoteichoic acid according to claim 2 wherein the induced inflammation is done by pro-inflammatorical co-treatment of the cancer, preferably by local pro-inflammatorical co-treatment of the cancer.

4. Lipoteichoic acid as free acid or as any pharmaceutically acceptable salt or hydrate thereof for use in the treatment of cancer after induced inflammation in the cancer.

5. The Lipoteichoic acid according to claim 4 wherein the induced inflammation is done by pro-inflammatorical co-treatment of the cancer, preferably by local pro-inflammatorical co-treatment of the cancer.

6. Lipoteichoic acid as free acid or as any pharmaceutically acceptable salt or hydrate thereof for use in the treatment of pro-inflammatorically co-treated cancer.

7. The Lipoteichoic acid according to any of claim 1 or 6, wherein the inflammation or the co-treatment inducing such inflammation is local.

8. The lipoteichoic acid according to any of claims 2 to 6, or claim 7 referring back only to claim 6, wherein the treatment is following while the cancer is in a state of inflammation or within 1 or 2 months, or within 1 or 2 weeks or within 1 week, or within 1 to 5 days, or within 1 to 3 days after induction of inflammation in the cancer or pro-inflammatorical co-treatment of the cancer.

9. The lipoteichoic acid according to any of claims 3, 5, 6, or 8, or claim 7 referring back only to claim 6, wherein the pro-inflammatorical co-treatment is done by radiation, preferably by radiation of the tissue comprising the cancer, preferably by X-ray radiation of the tissue comprising the cancer; with preferably this radiation co-treatment being done previous to the treatment with lipoteichoic acid.

10. The lipoteichoic acid according to any of claims 3, 5, 6, 8 or 9, or claim 7 referring back only to claim 6, wherein the pro-inflammatorical co-treatment is done by, optionally previous to the treatment, application of at least one pro-inflammatory substance, preferably is done by, optionally previous to the treatment, application of at least one pro-inflammatory substance to the tissue comprising the cancer and/or is done by, optionally previous to the treatment, injection of at least one pro-inflammatory substance, more preferably is done by, optionally previous to the treatment, injection of at least one pro-inflammatory substance to the tissue comprising the cancer.

11. The lipoteichoic acid according to claim 10, wherein the pro-inflammatory substance is selected from pro-inflammatory interleukins.

12. The lipoteichoic acid according to any of claims 1 to 11, wherein the treatment with lipoteichoic acid is done by injection, including, s.c., i.m., i.v., i.a., intra-ocular, intra-pleural, intra-abdominal, or intra-thecal injection.

13. The lipoteichoic acid according to any of claims 1 to 12, wherein the treatment with lipoteichoic acid is done by applying an amount of 0.1 to 10 µmol, 0.5 to 2.0 µmol or 1 µmol lipoteichoic acid applied in one or several applications per day.

14. The Lipoteichoic acid according to any of claim 1 to 13 comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid;
preferably wherein the glycolipid is a glyceroglycolipid in which one or more mono- or oligosaccharids are bound via a glycosidic bond to a lipid molecule consisting of fatty acids being bound by an ester bond to the glycerol;
more preferably wherein the glyceroglycolipid comprises a diacylglycerol with two fatty acid chains of 10 to 20 carbon atoms each being bound by an ester bond to the glycerol, and/or wherein the fatty acid chains being linear or branched and non-substituted and/or having a length of 10 to 20 carbon atoms each, most preferably of 12, 14, 16 or 18 carbon atoms.

15. The Lipoteichoic acid according to any of claims 1 to 13 being a structure according to general formula I 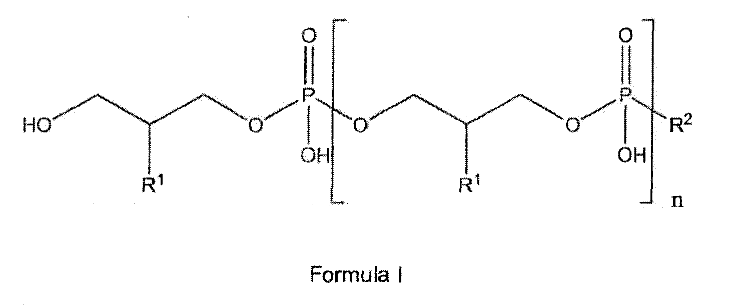 wherein
the mean value of n is 8 to 40,
R¹ is either OH or O-D-alanyl and
R² is
either 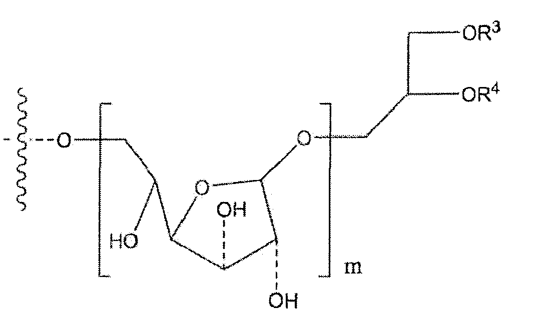 or its tautomeric equivalent 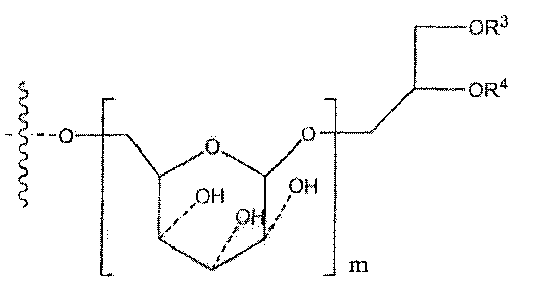 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures;
preferably being a structure according to general formula I 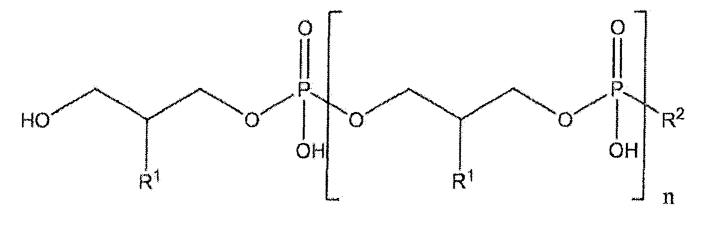 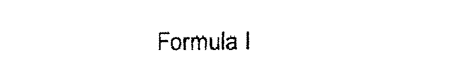 wherein
the mean value of n is 9,
R¹ is either OH or O-D-Alanyl and R² is 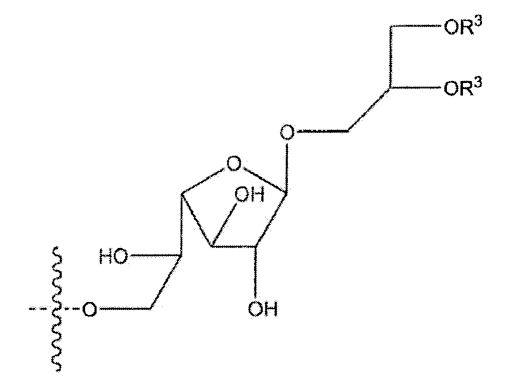 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures;
**or**
being a purified lipoteichoic acid isolated or isolatable from the Streptococcus sp. PT strain DSM 8747, preferably containing a beta-galactofuranosyl(1-3)glycerol-di-ester moiety;
**or**
being a compound according to any of general formulas Ia, Ib, lc, or Id 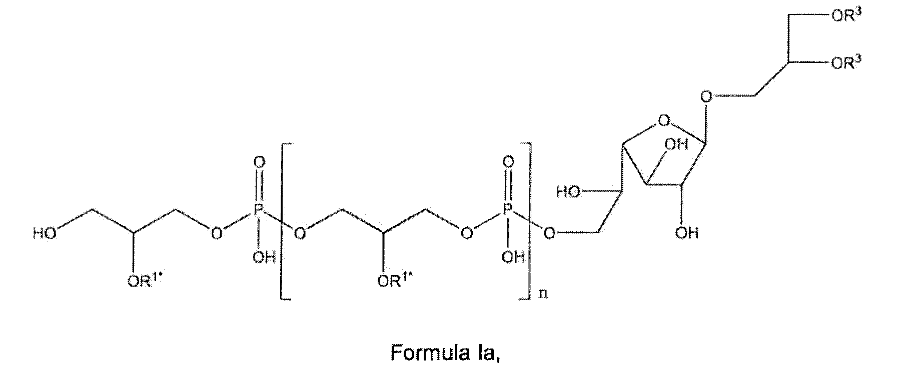 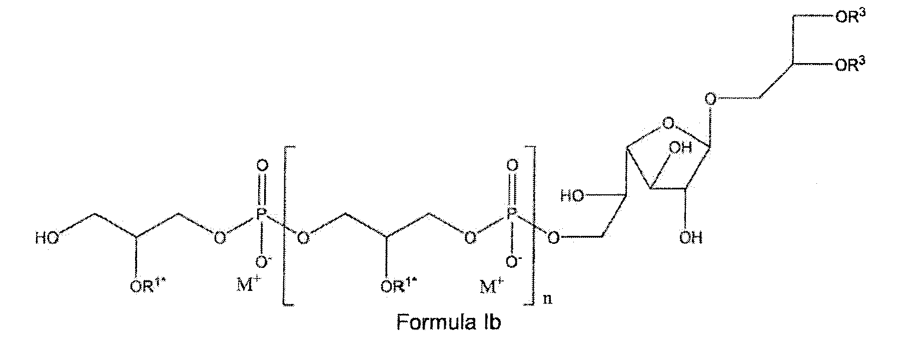 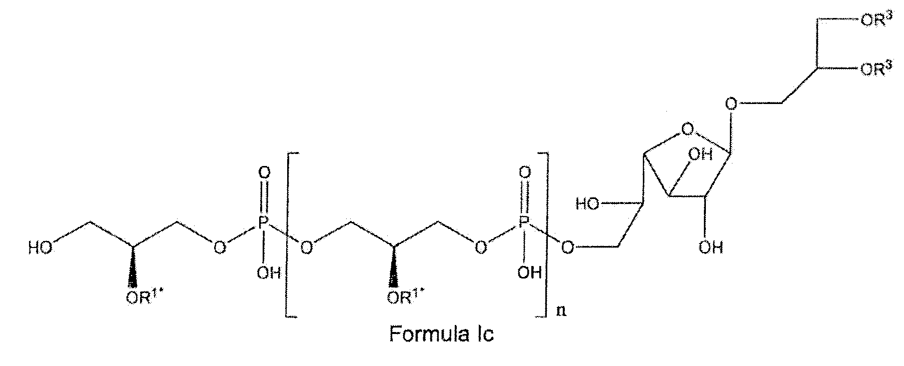 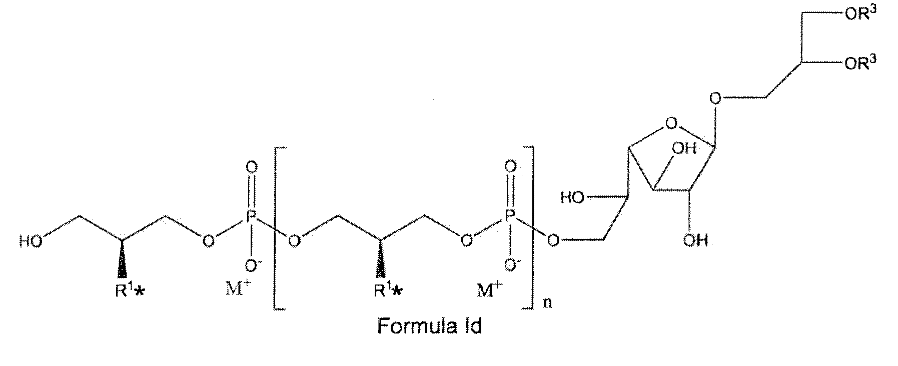 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion;
**most preferably**
being LTA-T as a free acid or as a pharmaceutically acceptable salt or hydrate thereof, especially being LTA-T as a free acid or as a sodium salt.
